(19) Europäisches Patentamt
European Patent Office
Office européen des brevets

(11) Publication number: 0 043 706
A2

# (12) EUROPEAN PATENT APPLICATION

(21) Application number: **81303024.4**

(22) Date of filing: **02.07.81**

(51) Int. Cl.³: **C 07 D 489/02**
// C07D217/20, C07D221/28, C07D491/10, A61K31/485, (C07D491/10, 317/00, 221/00)

(30) Priority: **03.07.80 US 165690**

(43) Date of publication of application: **13.01.82 Bulletin 82/2**

(84) Designated Contracting States: **BE CH DE FR GB LI NL SE**

(71) Applicant: **THE UNITED STATES OF AMERICA represented by the Secretary U.S. Department of Commerce, National Technical Information Service Office of Government Inventions and Patents 5285 Port Royal Road, Springfield, Virginia 22161 (US)**

(72) Inventor: **Rice, Kenner Cralle, 4704 Red Fox Road, Rockville Maryland 20852 (US)**

(74) Representative: **Woodman, Derek et al, FRANK B. DEHN & CO. Imperial House 15-19 Kingsway, London WC2B 6UZ (GB)**

## (54) A short total synthesis of dihydrothebainone, dihydrocodeinone, and nordihydrocodeinone.

(57) A method of producing morphinan compounds which incorporates double advantages over the prior art, such as the utilization of β, γ-unsaturated ketones in place of the α,β-unsaturated ketones previously used. Additionally, in the step where β,γ-unsaturated bromoketones (13 or 14) proceed to 1-bromo-N-formylnordihydrothebainone (morphinan) (17) there are utilized super acids, such as trifluoromethane sulfonic, trifluoroethane sulfonic and mixtures thereof, and also antimony pentafluoride and mixtures of hydrogen fluoride and antimony pentafluoride. Super acids work, whereas the prior art shows that cyclization in the presence of acids, even strong acids, such as sulfuric, phosphoric, do not work.

EP 0 043 706 A2

# A Short Total Synthesis of Dihydrothebainone, Dihydrocodeinone, and Nordihydrocodeinone

The present application relates to compounds which may be described as morphine agonists and antagonists. The synthesis utilized is capable of producing all of the medically important opium derivatives of the morphinan type, including thebaine.

Among the advantages emphasized by the present invention are the utilization of $\beta,\gamma$-unsaturated ketones where in the past there have been used $\alpha,\beta$-unsaturated ketones. Secondly, in the step below where $\beta,\gamma$-unsaturated bromoketones (13 or 14) proceed to 1-bromo-N-formylnordihydrothebainone (morphinan) (17), in this process there are utilized super acids, such as trifluoromethane sulfonic, trifluoroethane sulfonic and mixtures thereof, and also antimony pentafluoride and mixtures of hydrogen fluoride and antimony pentafluoride. An additional advantage of the present process is that oxide bridge closure is accomplished in the N-nor series bromonordihydrothebainone (18)⟶ nordihydrocodeinone (21), thus affording ready access to either N-nor or N-methyl derivatives from the same intermediate 18. N-nor derivatives are of paramount importance in the synthesis of narcotic antagonists and the agonist-antagonist drugs. The N-methyl derivative, dihydrocodeinone (22), is a key intermediate which can be converted by established methods to codeine,

morphine, thebaine and all medically valuable opium derivatives of the morphinan type.

In this specification, morphine-type means alkaloid compounds generally of the morphinan structure. Specially interesting are morphine and morphinan agonist and antagonists.

Grewe codeine method is a ring closure method utilizing bromine as a blocking group and deactivating influence on the phenolic ring (cf. J. Het. Chem., June 1974, 363).

Birch reduction includes reduction with ammonia or lower amine and lithium (preferred) or other alkali metal.

The introduction of bromine in a 1:1 ratio followed by alkali metal base served to close the oxide bridge. In a process proceeding from bromonordihydrothebainone (18) the bridge head nitrogen may be alkylated using an acid aldehyde, a lower aldehyde, or ketone such as theyl aldehyde or acetone.

The chart below is a synopsis of the sequence of steps.

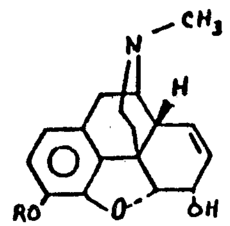

1: R=$CH_3$

2: R=H

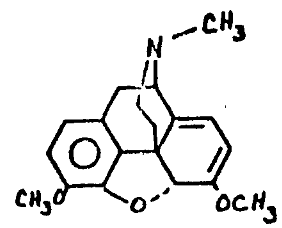

3

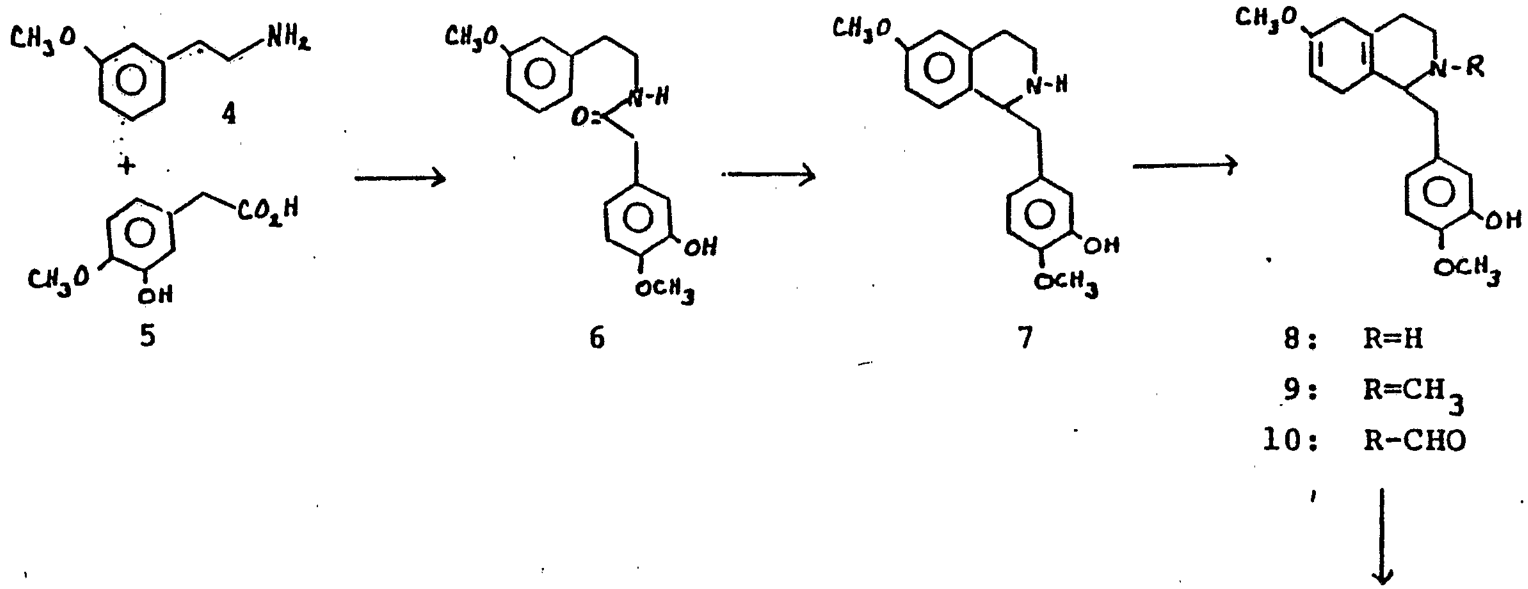

4

5

6

7

8: R=H

9: R=$CH_3$

10: R-CHO

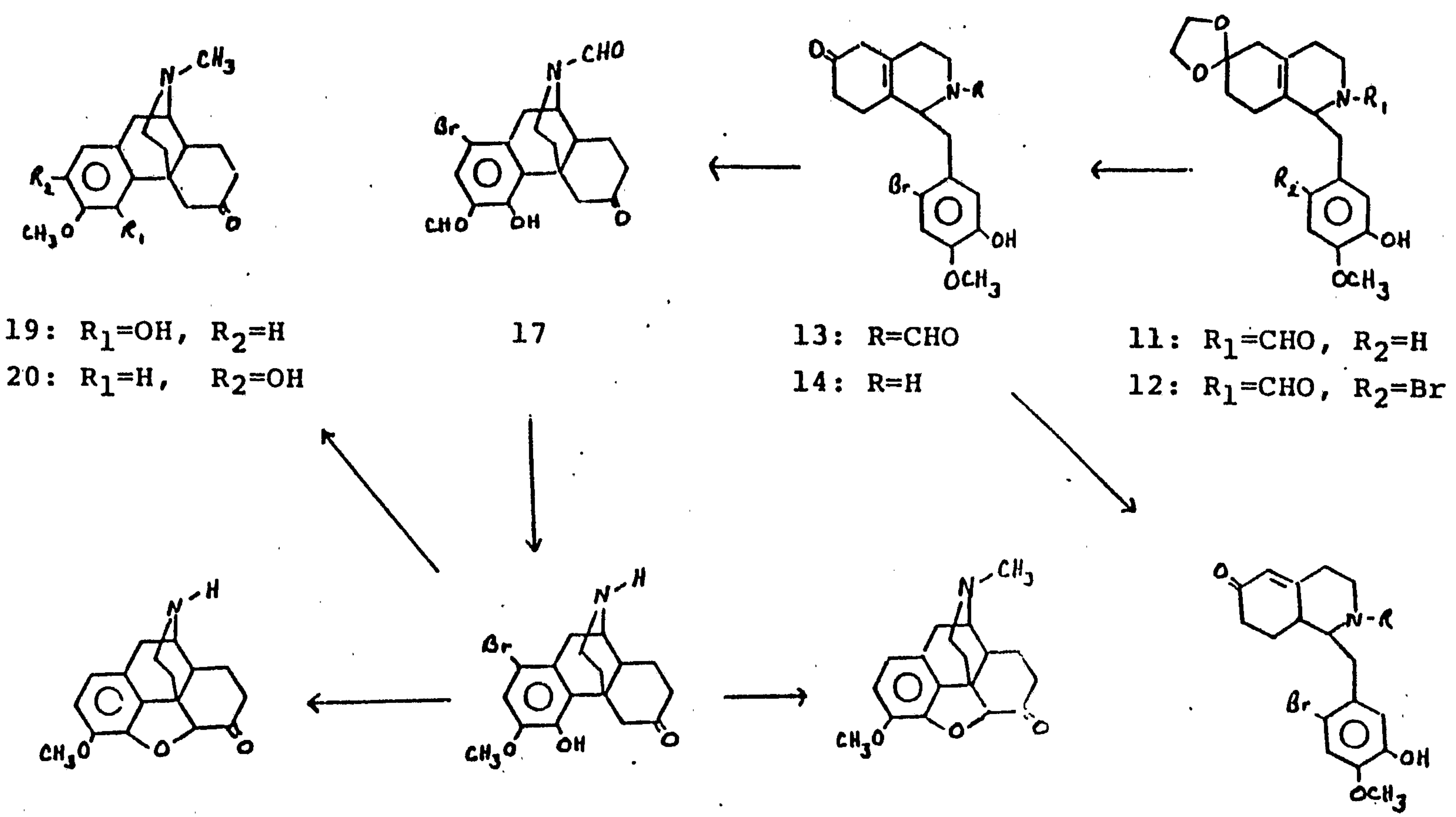

19: $R_1$=OH, $R_2$=H
20: $R_1$=H, $R_2$=OH

17

13: R=CHO
14: R=H

11: $R_1$=CHO, $R_2$=H
12: $R_1$=CHO, $R_2$=Br

21

18

22

15: R=H
16: R=CHO

Prior Art Statement

British Patent 1,330,581 and Netherlands Patent 7,107,921 - Any pertinency of these two patents is diminished by published studies [Beyerman et al, Recl. Trav. Chim. Pays-Bas, 95:184 (1976), and DeGraw et al, J. Heterocyclic Chem., 11:363 (1974)]. These two Merck patents claim Grewe cyclization to N-acyl derivatives of morphinan 18. The present invention shows that only $\beta,\gamma$-unsaturated bromoketone will operate as precursors for the morphinan carbon-nitrogen skeleton.

DeGraw et al, J. Heterocyclic Chem., 11:363 (1974), see page 367, footnote 4.

Beyerman et al, Recl. Trav. Chim., 95:184 (1976), see page 2, column 1.

Birch et al, "Reduction by Metal-Ammonia Solution and Related Reagents," Advances in Organic Chemistry, Vol. 8, Wiley-Interscience, 1972, pages 1-65.

May et al, "Morphine and Its Modifications," Medicinal Chemistry, Vol. 5, D. Stevens, Analgesics, 1965, pages 123-174.

International Series of Monographs in Organic Chemistry, Vol. 8, Synthetic Analgesics, Part 2-A, Morphinans, Pergamon Press, 1966, pages 1-104.

With reference to the use of super acids, the invention encompasses such acids as trifluoromethane sulfonic, trifluoroethane sulfonic, etc., and it is maintained that the use of these very strong acids overcomes the difficulty of use of acids, such as sulfuric, phosphoric, etc.

Relative to the compounds produced by alkylating the nitrogen bridge head, this reaction will work with lower amines and lower ketones. The utility of the products akin to nordihydrocodeinone (21) with N-alkylate are morphine agonists or antagonists. These compounds with the N-alkylate structure are denoted as tertiary amines and specially where N-methyl, N-ethyl, etc., are produced and the alkyl chain is $C_1$-$C_6$. Of the ketones, acetone and methyl ethyl ketone will operate. They are regarded as quite important for morphine overdose.

In general, as to utility, dihydrothebainone (19) is noted in J. Med. Chem., 19:1171 (1976) and dihydrocodeinone (22) is a prescription drug on the market at present produced by Mallinckrodt, etc., as Hydrocodone.

As a general summary of the above chart, the following general description in made commencing with codeine (1).

Racemic dihydrothebainone (19), nordihydrocodeinone (21) and dihydrocodeinone (22) were synthesized in high overall yield from 3-methoxyphenethylamine (4), via the key intermediate (±)-1-bromonordihydrothebainone (18); the route utilized unprotected phenolic intermediates, involved directed Grewe-type cyclization and for 21 and 22, exploited novel oxide bridge closure in the N-nor series.

Natural (-)-codeine (1) continues to occupy a position of central importance among the medically valuable derivatives of the opium poppy as the most frequently prescribed analgesic-antitussive agent worldwide. Since the first total synthesis of (-)-codeine (1) and (-)-morphine (2), other successful routes, including Grewe-type and biomimetic approaches, have appeared. However, a practical total synthesis of these drugs has remained elusive. These and continuing efforts, together with possible shortages, underscore the desirability of securing a route which could render licit production of medical opiates independent of the natural and sole commercial source of these drugs. Since the reports that Grewe-type electrophilic cyclization of (±)-1-benzylhexahydroisoquinoline (9) afforded a 3% yield of the codeine precursor dihydrothebainone (19) (with isomeric 20 as the vastly predominant cyclization product), several groups have attempted to utilize this approach to codeine by introduction of a blocking substituent at the 1-position of the benzyl moiety in order to direct cyclization to the desired dihydrothebainone oxygenation pattern. Studies utilizing a 1-methyl substituent were successful in this regard. However, such an approach must also employ a readily removable group to be of value in synthesis of codeine and congeners, of course, not the case in the 1-methyl series. Substitution of bromine for methyl, unsuccessful hithertofore, would be ideal, since transformation of 4-hydroxymorphinans such as 19 to 22 (with the oxide bridge closed as in codeine) first involves bromination at C-1 of the morphinan system and later removal of the C-1 bromine atom by hydrogenolysis. Recent work describing conversion of (-)-dihydrothebainone [(-)-19] to (-)-codeine (1) (68% overall), via

(-)-dihydrocodeinone, [(-)-22] and to (-)-thebaine (3), (an important minor opium alkaloid) in somewhat higher yield, renders any totally synthetic approach yielding 1-bromodihydrothebainone derivatives still more attractive. Such an approach which utilizes unprotected phenolic intermediates is short, experimentally simple, and affords (±)-dihydrothebainone (19), (±)-nordihydrocodeinone (21) and (±)-dihydrocodeinone (22) in high overall yield via the key intermediate (±)-1-bromonordihydrothebainone (18) is included in this disclosure. The sequence rests essentially on high yield preparation of β,γ-unsaturated bromoketone 13, that is converted by directed Grewe-type cyclization into (±)-1-bromo-N-formylnordihydrothebainone (17), and on the novel oxide bridge closure in the N-nor series, which optionally provides ready access to either N-methyl or N-nor derivatives.

Heating a mixture of amine 4 and pure acid 5 at 200°C for 2 h under argon afforded amide 6 (95%, EtOAc). Cyclization of 6 (0.35 mol scale) essentially as described for preparation of the 7-OH derivative of 7, generated an aqueous solution of the 1,2-dehydro derivative of 7. Neutralization to pH 4-5 with concentrated aqueous $NH_3$ and reduction with an equimolar quantity of $NaCNBH_3$ in refluxing 45% MeOH (final concentration) for 1.5 h afforded pure (TLC) 7 (86%), mp 199.5-201.5°C. Birch reduction with lithium and ammonia of 85 mmol of unpurified 7 with 1.92 g atom of lithium in 450 ml of liquid $NH_3$, 225 mL each of dry THF and t-BuOH at -55 to -65°C for 4 h, then at -75°C until no 7 remained by TLC 9 ~1.5 h) afforded (90%) essentially

pure (TLC) 8, mp 179.5-181.5°C. Refluxing unpurified 8 with 1.5 equivalents of pure PhOCHO in 10 volumes of EtOAc until homogeneous then ~0.75 h until TLC showed absence of 8 gave (94%) pure 10, mp 141-143°C ($Et_2O$-PhOH) which, like the N-formyl derivatives described below and others, existed as two distinct rotomers, as shown by NMR. Stirring a solution of 10 (25°C, 1 h) in 20 volumes of dry THF containing 1% (v/v) $CH_3SO_3H$ and 3 molar equivalents of ethylene glycol generated a solution of ketal 11 (quantitatively by TLC) which was treated at 0-5°C during 0.5 h with 1.05 equivalents of recrystallized N-bromoacetamide (NBA) to afford essentially pure bromoketal 12 after neutralization with $NH_3$ gas, solvent evaporation and workup with $CHCl_3$-$H_2O$. Bromoketal 12, mp 182.5-184°C, (EtOAc) could be readily isolated in 88% yield but was most efficiently deketalized in 6 volumes of 5:1 88% $HCO_2H$-$H_2O$ (25°C, 1 h) followed by $CHCl_3$-aqueous $NaHCO_3$ workup to afford 13, mp 203.5-206.5°C (DMF-EtOAc), IR ($CHCl_3$) 1717 (C=O) and 1665 (NCHO) cm,$^{-1}$ in 90% yield from 10. Bromoketone 13 underwent Grewe-type cyclization to (±)-1-bromo-N-formylnordihydrothebainone (17), mp 229.5-231.5°C (CMF-$H_2O$) in 60% isolated yield when treated in a screw-capped, high density polyethylene bottle with 14% $NH_4F \cdot HF$ in dry $CF_3SO_3H$ (0°C, under argon for 72-96 h until 13 had essentially disappeared by TLC). Also, isomerization of 13 occurred to give (TLC) the α,β-unsaturated bromoketone 16. Pure 16 afforded (TLC) only traces of morphinan 17 and β,γ-unsaturated ketone 13 under the conditions used to cyclize 13 to 17. Treatment of pure morphinan 17 under these conditions gave no 13 or 16 (TLC); the acid catalyzed equilibrium of 13 and 16 lies nearly exclusively toward the side of the latter,

which undergoes little, if any, morphinan cyclization under these conditions. Refluxing isolated 17 in 10:1 MeOH-37% aqueous HCl for 18 h afforded (±)-1-bromonordihydrothebainone (18), mp 220-223°C which was easily isolated in 92% yield by workup with $NH_3$-$H_2O$-isopropanol. (±)-Dihydrothebainone (19), mp 173-175°C, was obtained directly and quantitatively from 17 by hydrolysis as above, evaporation to dryness, and hydrogenation of the residue in 2N AcOH containing 50 mg 10% Pd/C, 0.3 mL of 37% HCHO and 5 mmol of NaOAc per mmol of 17, followed by workup with $CHCl_3$-aqueous $NH_3$. Bromination (1.1 mol of $Br_2$, 25°C, 2h) of an AcOH solution of the dry residue from hydrolysis of 17, evaporation, treatment of the residue with $CHCl_3$-1N NaOH and hydrogenation as above without addition of HCHO afforded an 80% yield (from 17) of (±)-nordihydrocodeinone (21), that was readily isolated (isopropanol, 1.1 equivalents of 37% aqueous HCl) as 21·HCl·0.5 $H_2O$, mp 292-295°C dec; anhydrous 21 base, mp 136.5-138°C ($PhCH_3$). This is the first example of closure of the oxide bridge in the basic N-nor series and is of potential interest in the synthesis of narcotic antagonists. When 17 was treated as in the preparation of 21, and 0.3 mL of 37% HCHO/mmol of 17 was added to the hydrogenation medium (±)-dihydrocodeinone (22), mp 158-160°C, was readily isolated as 22·TsOH, mp 248-251°C in 79% yield from 17.

This straightforward total synthesis of (±)-dihydrothebainone (19), (±)-nordihydrocodeinone (21), and (±)-dihydrocodeinone (22) in 37, 30 and 29% overall yields respectively, from readily available 3-methoxyphenethylamine (4) requires isolation of only 6 intermediates. These are directly obtained sufficiently pure

for further transformation. In view of these results, the high-yielding conversion of (-)-19 to (-)-thebaine (3) and (-)-codeine (1) discussed above and the facile O-demethylation of the latter to (-)-morphine (2), a practical total synthesis of these alkaloids (and the thebaine based drugs) has resulted.

Resolution of racemic tetrahydroisoquinoline 7 is accomplished by formation and fractional crystallization of diasterisomeric salts with optically active acids such as tartaric, mandelic and tartranilic acid etc. The appropriate optically active base 7 is then regenerated and subjected the reaction sequence described herein to afford morphinan derivatives with the natural morphine absolute configuration.

CLAIMS:

1. A sequential method of preparing morphine-type agonists and antagonists which comprises commencing with an acid and an amine to form an amide proceeding through a ring closure with $\beta,\gamma$-unsaturated ketones and utilizing a super acid to produce morphinan and subsequently producing 1-bromonordihydrothebainone, nordihydrocodeinone, and dihydrocodeinone.

2. In a method of preparing morphine-type agonists and antagonists which used $\alpha,\beta$-unsaturated ketones, the step which consists of advantageously using $\beta,\gamma$- unsaturated ketones.

3. A process of producing morphine-type agonists and antagonists including the utilization of acid in a ring closure step to morphinan, the step which comprises utilizing in place of acids a super acid selected from the group consisting of trifluoromethane sulfonic acid, trifluoroethane sulfonic acid and mixtures thereof, and antimony pentafluoride and mixtures of hydrofluoride antimony pentafluoride.

4. The method according to Claim 1 in which morphinan is reacted to form 1-bromonordihydrothebainone which, in turn, optionally produces nordihydrocodeinone and dihydrocodeinone.

5. The method according to Claim 1 wherein in one step the ketal is brominated to bromoketal (ketal $\longrightarrow$ bromoketal) followed later by a reaction which is parametered by introduction of bromine in 1:1 ratio followed by alkali metal base which closes the oxide bridge.

6. The method according to Claim 1 wherein the bridge nitrogen is alkylated by a compound selected from $C_1$-$C_6$ aldehyde and lower ketone.